(19) Europäisches Patentamt
European Patent Office
Office européen des brevets

(11) **EP 4 718 124 A1**

(12) **EUROPEAN PATENT APPLICATION**

(43) Date of publication:
**01.04.2026  Bulletin 2026/14**

(21) Application number: **25204327.8**

(22) Date of filing: **24.09.2025**

(51) International Patent Classification (IPC):
*G01T 7/00* $^{(2006.01)}$   *G05F 1/46* $^{(2006.01)}$
*G05F 1/56* $^{(2006.01)}$   *G05F 1/565* $^{(2006.01)}$
*G05F 1/575* $^{(2006.01)}$   *G05F 3/26* $^{(2006.01)}$
*H03G 3/30* $^{(2006.01)}$

(52) Cooperative Patent Classification (CPC):
**H03G 3/3015; G01T 1/17; G05F 1/462;
G05F 1/463; G05F 1/467; G05F 1/561; G05F 1/565;
G05F 1/575; G05F 3/26; H03F 3/45183;**
H03F 2200/102; H03F 2203/21148

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **26.09.2024  US 202418897255**

(71) Applicant: **Avago Technologies International
Sales
Pte. Limited
Singapore 768923 (SG)**

(72) Inventors:
• **Enne, Reinhard**
**3202 Hofstetten-Grünau (AT)**
• **Gaberl, Wolfgang**
**1220 Vienna (AT)**
• **Davidovic, Milos**
**1110 Vienna (AT)**

(74) Representative: **Dilg, Haeusler, Schindelmann
Patentanwaltsgesellschaft mbH
Leonrodstraße 58
80636 München (DE)**

(54) **SYSTEMS AND METHODS FOR SENSOR BASELINE DETECTION AND COMPENSATION**

(57)    The subject technology is directed to systems and methods for sensor baseline detection and compensation. In an embodiment, the subject technology provides an apparatus comprising a first amplifier configured to receive a current signal and generate a first voltage signal associated with the current signal. The apparatus also comprises a first circuit configured to generate a second voltage signal based on the first voltage signal. The first circuit further comprises a switch configured to adjust the second voltage signal in response to changes in the first voltage signal, and a control circuit configured to control the rate of adjustment of the second voltage signal. There are other embodiments as well.

**Figure 1**

**Description**

FIELD OF INVENTION

The subject technology is directed to systems and methods for imaging technologies.

BACKGROUND OF THE INVENTION

In a wide range of electronic systems, sensors play an important role in detecting and measuring various physical phenomena, such as light, temperature, pressure, and radiation. These sensors convert physical inputs into electrical signals that can be processed and analyzed for a variety of applications. For example, in medical imaging systems, advanced technologies like computed tomography (CT) and positron emission tomography (PET) rely on highly sensitive sensors to detect incoming photons and convert them into electrical signals that represent the photon flux. These sensors may be coupled to application-specific integrated circuits (ASICs) that handle the sensor outputs, process the signals, and extract meaningful data. The quality and accuracy of the imaging process are directly influenced by the performance of these sensors and their associated front-end circuits.

One of the challenges in these systems is maintaining a stable reference level, commonly referred to as the baseline, in the sensor output signals. The baseline represents a steady-state or direct current (DC) component of the signal in the absence of significant input changes. It may serve as a reference point for detecting fluctuations and variations in the sensor output. However, the baseline is susceptible to fluctuations caused by various factors, including temperature changes, cosmic rays, aging components, variations in the radiation source, and environmental noise. These fluctuations can lead to inaccuracies in signal interpretation, ultimately affecting the imaging quality and reliability.

Various approaches for optimizing baseline detection and compensation have been explored, but they have proven to be insufficient. It is important to recognize the need for new and improved systems and methods for baseline management in sensor-based applications.

BRIEF DESCRIPTION OF THE DRAWINGS

A further understanding of the nature and advantages of particular embodiments may be realized by reference to the remaining portions of the specification and the drawings, in which like reference numerals are used to refer to similar components. In some instances, a sub-label is associated with a reference numeral to denote one of multiple similar components. When reference is made to a reference numeral without specification to an existing sub-label, it is intended to refer to all such multiple similar components.

Figure 1 is a simplified diagram illustrating a front-end signal processing system according to embodiments of the subject technology.

Figure 2 is a simplified diagram illustrating a system for baseline detection and compensation according to embodiments of the subject technology.

Figure 3 is a simplified diagram illustrating a front-end signal processing system according to embodiments of the subject technology.

Figure 4 is a simplified diagram illustrating a system for baseline detection according to embodiments of the subject technology.

Figure 5 is a simplified diagram illustrating a system for baseline detection according to embodiments of the subject technology.

Figure 6 is a simplified diagram illustrating a system for baseline detection according to embodiments of the subject technology.

Figure 7 is a simplified diagram illustrating a system for baseline detection according to embodiments of the subject technology.

Figure 8 is a simplified diagram illustrating a system for baseline detection according to embodiments of the subject technology.

Figure 9 is a simplified diagram illustrating a system for baseline detection according to embodiments of the subject technology.

Figure 10 is a simplified diagram illustrating a system for baseline detection according to embodiments of the subject technology.

Figure 11 is a simplified diagram illustrating a system for baseline detection according to embodiments of the subject technology.

DETAILED DESCRIPTION OF THE INVENTION

The subject technology is directed to systems and methods for sensor baseline detection and compensation. In an embodiment, the subject technology provides an apparatus comprising a first amplifier configured to receive a current signal and generate a first voltage signal associated with the current signal. The apparatus also comprises a first circuit configured to generate a second voltage signal based on the first voltage signal. The first circuit further comprises a switch configured to adjust the second voltage signal in response to changes in the first voltage signal, and a control circuit configured to control the rate of adjustment of the second voltage signal. There are other embodiments as well.

As previously noted, baseline stability is important in sensor-based systems, where even minor fluctuations can lead to inaccuracies in signal interpretation. The term "baseline" may refer to a steady-state component of the signal that serves as a reference point for detecting variations. The baseline may be affected by various environmental factors, including temperature variations, cosmic rays, and changes in the radiation source's amplitude. These influences can cause the baseline to drift over time, particularly within the low-frequency range (e.g., within sub-kHz spectrum).

Some approaches for addressing baseline fluctuations involve the use of static digital-to-analog converters (DACs) to compensate for any shifts. However, these approaches often fail in dynamic environments where the baseline rapidly changes due to fluctuating operational conditions. Moreover, the process of detecting and correcting baseline shifts can introduce periods of inactivity, commonly referred to as blind time, during which the system is unable to process incoming signals. This blind time is particularly detrimental in continuous operation systems like CT and PET scanners, where even brief interruptions can degrade image quality and diagnostic accuracy.

In various embodiments, the subject technology provides a system for detecting and adjusting the baseline of sensor signals in real time, ensuring stable and accurate signal processing even in the presence of environmental fluctuations. By continuously tracking and compensating for baseline fluctuations, the system ensures accurate signal interpretation without the need for manual recalibration or introducing periods of system inactivity, thereby enhancing the reliability and efficiency of electronic systems.

The following description is presented to enable one of ordinary skill in the art to make and use the invention and to incorporate it in the context of particular applications. Various modifications, as well as a variety of uses in different applications, will be readily apparent to those skilled in the art, and the general principles defined herein may be applied to a wide range of embodiments. Thus, the subject technology is not intended to be limited to the embodiments presented but is to be accorded the widest scope consistent with the principles and novel features disclosed herein.

In the following detailed description, numerous specific details are set forth in order to provide a more thorough understanding of the subject technology. However, it will be apparent to one skilled in the art that the subject technology may be practiced without necessarily being limited to these specific details. In other instances, well-known structures and devices are shown in block diagram form, rather than in detail, in order to avoid obscuring the subject technology.

The reader's attention is directed to all papers and documents which are filed concurrently with this specification and which are open to public inspection with this specification, and the contents of all such papers and documents are incorporated herein by reference. All the features disclosed in this specification, (including any accompanying claims, abstract, and drawings) may be replaced by alternative features serving the same, equivalent, or similar purpose, unless expressly stated otherwise. Thus, unless expressly stated otherwise, each feature disclosed is one example only of a generic series of equivalent or similar features.

Furthermore, any element in a claim that does not explicitly state "means for" performing a specified function, or "step for" performing a specific function, is not to be interpreted as a "means" or "step" clause as specified in 35 U.S.C. Section 112, Paragraph 6. In particular, the use of "step of" or "act of" in the Claims herein is not intended to invoke the provisions of 35 U.S.C. 112, Paragraph 6.

When an element is referred to herein as being "connected" or "coupled" to another element, it is to be understood that the elements can be directly connected to the other element or have intervening elements present between the elements. In contrast, when an element is referred to as being "directly connected" or "directly coupled" to another element, it should be understood that no intervening elements are present in the "direct" connection between the elements. However, the existence of a direct connection does not exclude other connections, in which intervening elements may be present.

When an element is referred to herein as being "disposed" in some manner relative to another element (e.g., disposed on, disposed between, disposed under, disposed adjacent to, or disposed in some other relative manner), it is to be understood that the elements can be directly disposed relative to the other element (e.g., disposed directly on another element), or have intervening elements present between the elements. In contrast, when an element is referred to as being "disposed directly" relative to another element, it should be understood that no intervening elements are present in the "direct" example. However, the existence of a direct disposition does not exclude other examples in which intervening elements may be present.

Similarly, when an element is referred to herein as being "bonded" to another element, it is to be understood that the elements can be directly bonded to the other element (without any intervening elements) or have intervening elements present between the bonded elements. In contrast, when an element is referred to as being "directly bonded" to another element, it should be understood that no intervening elements are present in the "direct" bond between the elements. However, the existence of direct bonding does not exclude other forms of bonding, in which intervening elements may be present.

Likewise, when an element is referred to herein as being a "layer," it is to be understood that the layer can be a single layer or include multiple layers. For example, a conductive layer may comprise multiple different conductive materials or multiple layers of different conductive materials, and a dielectric layer may comprise multiple dielectric materials or multiple layers of dielectric materials. When a layer is described as being coupled or connected to another layer, it is to be understood that the coupled or connected layers may include intervening elements present between the coupled or connected layers. In contrast, when a layer is referred to as being "directly" connected or coupled to another layer, it should be understood that no intervening elements are present between the layers. However, the existence of directly coupled or connected layers does not exclude other connections in which intervening elements may be present.

Moreover, the terms left, right, front, back, top, bottom, forward, reverse, clockwise and counterclockwise are used for purposes of explanation only and are not limited to any fixed direction or orientation. Rather, they are used merely to indicate relative locations and/or directions between various parts of an object and/or components.

Furthermore, the methods and processes described herein may be described in a particular order for ease of description. However, it should be understood that, unless the context dictates otherwise, intervening processes may take place before and/or after any portion of the described process, and further various procedures may be reordered, added, and/or omitted in accordance with various embodiments.

Unless otherwise indicated, all numbers used herein to express quantities, dimensions, and so forth should be understood as being modified in all instances by the term "about." In this application, the use of the singular includes the plural unless specifically stated otherwise, and use of the terms "and" and "or" means "and/or" unless otherwise indicated. Moreover, the use of the terms "including" and "having," as well as other forms, such as "includes," "included," "has," "have," and "had," should be considered non-exclusive. Also, terms such as "element" or "component" encompass both elements and components comprising one unit and elements and components that comprise more than one unit, unless specifically stated otherwise.

As used herein, the phrase "at least one of" preceding a series of items, with the term "and" or "or" to separate any of the items, modifies the list as a whole, rather than each member of the list (i.e., each item). The phrase "at least one of" does not require the selection of at least one of each item listed; rather, the phrase allows a meaning that includes at least one of any one of the items, and/or at least one of any combination of the items. By way of example, the phrases "at least one of A, B, and C" or "at least one of A, B, or C" each refer to only A, only B, or only C; and/or any combination of A, B, and C. In instances where it is intended that a selection be of "at least one of each of A, B, and C," or alternatively, "at least one of A, at least one of B, and at least one of C," it is expressly described as such.

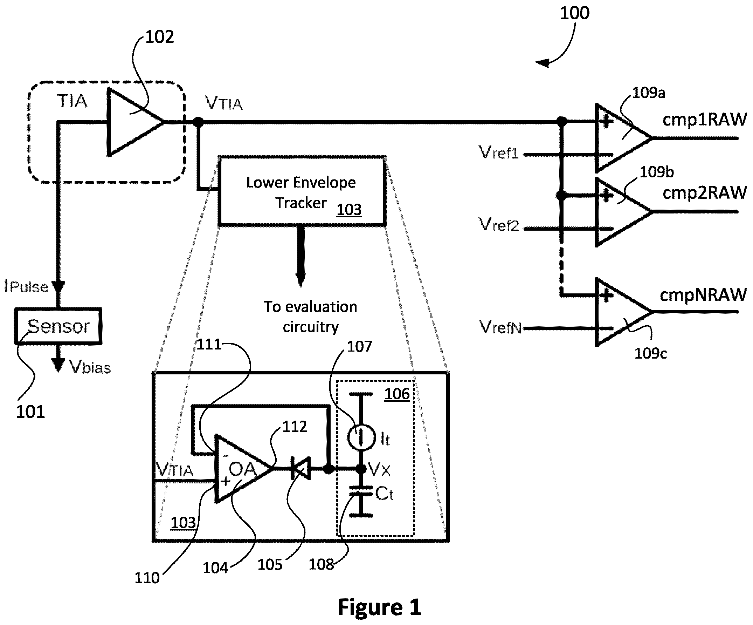

Figure 1 is a simplified diagram illustrating a front-end signal processing system 100 according to embodiments of the subject technology. This diagram merely provides an example, which should not unduly limit the scope of the claims. One of ordinary skill in the art would recognize many variations, alternatives, and modifications.

In various implementations, system 100 may be part of a sensor-based system designed to receive, process, and/or interpret signals generated by various types of sensors and may be used in various applications such as medical imaging, industrial monitoring, environmental sensing, or telecommunications. For instance, system 100 can be utilized in applications requiring high precision and real-time processing, such as CT and PET scanners, where accurately detecting and tracking baseline shifts in sensor signals is beneficial for producing high-quality diagnostic images. Other applications may include optical or radiation detectors, automotive systems (e.g., LiDAR), and scientific instruments that rely on sensitive signal detection and processing.

As shown, system 100 may include sensor 101. For instance, the term "sensor" may refer to a device that detects or measures physical properties (e.g., light, temperature, radiation, pressure, or other environmental phenomena) and converts them into electrical signals. Examples of sensors may include, without limitation, photodiodes, thermocouples, pressure sensors, radiation detectors, and/or the like. In some examples, sensor 101 may include a photon detector, which is configured to detect incoming photons and convert the resulting energy into an electrical current signal (e.g., $I_{Pulse}$) that is proportional to the photon flux. This current signal may carry event-driven information, such as the detection of individual photons or groups of photons in medical imaging systems.

In some embodiments, the output current signal of sensor 101 may include an alternating current (AC) component and/or a direct current (DC) component. For instance, the AC component may represent the photon detection events, where the intensity and timing of the signal vary based on the number and energy of the detected photons. The DC component-which may be referred to as baseline-may represent the steady-state portion of the signal when no significant external events or photon detection occurs. In practice, the baseline value can fluctuate over time due to factors such as temperature variations, environmental changes, or component aging. The baseline serves as a reference level for event detection, and maintaining its stability is beneficial to ensure that genuine photon events (e.g., AC component) are accurately detected and interpreted. If the baseline shifts, noise or baseline drift may be mistaken for actual events, reducing the accuracy of system 100.

In some implementations, system 100 may include first amplifier 102. For instance, first amplifier 102 may include a transimpedance amplifier (TIA). The term "transimpedance amplifier" may refer to an electronic device that converts input current to a corresponding output voltage. Examples of TIAs may include, without limitation, photodiode TIAs, low-noise TIAs, programmable gain TIAs, and/or the like. First amplifier 102 may be configured to receive a current signal and generate a first voltage signal. The first voltage signal may be associated with the current signal. For instance, first amplifier 102 is configured to convert the current signal $I_{Pulse}$ generated by sensor 101 into a first voltage signal (e.g., $V_{TIA}$). The first voltage signal may be proportional to the magnitude of the current detected by sensor 101 and represents the photon flux or other physical phenomena being measured.

In various embodiments, system 100 may include first circuit 103 coupled to first amplifier 102. First circuit 103 may be configured to detect a reference level of the first voltage signal. The term "reference level" may refer to a voltage or signal threshold that is used as a baseline or comparison point for detecting variations in the signal. The reference level may represent a steady-state value associated with the first voltage signal when no significant external events or fluctuations occur. For the purposes of this disclosure, the terms "reference level" and "baseline" may be used interchangeably. In some examples, first circuit 103 may be configured to track the baseline of the output signal generated by first amplifier 102. For instance, first circuit 103 incrementally tracks the envelope of the first voltage signal, focusing on the portion of the signal that remains unaffected by transient events, such as photon detection or other signal fluctuations. When events occur that cause an increase in the signal (e.g., an increase in $V_{TIA}$ due to a photon detection) circuit 103 targets the lower envelope of the signal. The lower envelope may refer to the lowest point of the signal over time, which represents the baseline that is isolated from event-related spikes. By isolating the baseline portion of the signal, the system can effectively differentiate between transient events and the baseline.

Conversely, when events cause a decrease in the signal, circuit 103 may be configured to monitor the upper envelope of the signal. The upper envelope may refer to the highest point of the signal over time, representing the upper boundary of the signal unaffected by downward fluctuations. For the purposes of this disclosure, it is assumed that the lower envelope will be tracked, and circuit 103 may be referred to as lower envelope tracker. It should be recognized that the specific implementation of upper or lower envelope tracking is non-limiting, and one of ordinary skill in the art would appreciate that various modifications, variations, and alternatives may be made without departing from the broader scope of this disclosure. Other envelope-tracking mechanisms may also be used, depending on the application or system requirements.

The lower envelope tracking circuit operates by continuously monitoring the baseline, preventing long-term drift due to external factors like temperature changes or components aging. In various implementations, first circuit 103 includes second amplifier 104. The term "amplifier" may refer to a device or circuit that increases the magnitude of an input signal. Examples of amplifiers may include operational amplifiers (op-amps), differential amplifiers, power amplifiers, and/or the like. For instance, second amplifier 104 may include an operational amplifier coupled to first amplifier 102. First circuit 103 may be configured to receive the first voltage signal (e.g., $V_{TIA}$) and generate a second voltage signal (e.g., $V_x$) based on the first voltage signal.

In some examples, circuit 103 may further include switch 105 coupled to second amplifier 104. The term "switch" may refer to an electronic component or device that controls the flow direction of current or voltage in a circuit. Examples of switches may include transistors, diodes, relays, and/or the like. Second amplifier 104 may include first input 110, second input 111, and output 112. First input 110 may be configured to receive the first voltage signal. Second input 111 may be configured to receive the second voltage signal (e.g., $V_x$). In some implementations, switch 105 may be coupled to output 112 of second amplifier 104 and serve as the control mechanism for a feedback loop within circuit 103. For instance, the feedback may be established by connecting output 112 to second input 111. In some examples, second amplifier 104 may output an intermediate signal to drive the system to adjust the second voltage signal (e.g., $V_x$) through the feedback loop. For instance, $V_x$ represents the lower envelope of the first voltage signal. In other words, circuit 103 isolates and amplifies the baseline portion of the signal, filtering out the event-driven (e.g., AC component) fluctuations, allowing the system to focus on the steady-state level that is unaffected by transient events.

In some embodiments, the feedback loop continuously compares the second voltage signal to the input signal (e.g., the first voltage signal), enabling circuit 103 to track the baseline and respond to any deviations in the first voltage signal. For instance, when the first voltage signal (e.g., $V_{TIA}$) falls below the second voltage signal (e.g., $V_x$), switch 105 may close the loop, allowing feedback to flow from output 112 back to input 111 of second amplifier 104. This feedback adjusts the second voltage signal, aligning it with the lower envelope of the first voltage signal. When the first voltage signal exceeds the second voltage signal, switch 105 may open and disable the feedback, preventing the second voltage signa from being affected by event-related spikes. As the signal stabilizes, the second voltage signal may gradually align with the first voltage signal in the positive direction, ensuring smooth baseline adjustments without abrupt changes.

According to some embodiments, circuit 103 further includes control circuit 106. The term "control circuit" may refer to a circuit that governs the operation or behavior of other components in a system. For instance, control circuit 106 may control the timing or speed of a specific operation (e.g., by controlling how fast or slow a signal is adjusted over time). In some examples, control circuit 106 may be configured to control a rate of adjustment of the second voltage signal. The rate of adjustment may refer to the speed at which the second voltage signal (e.g., $V_x$) can change in response to fluctuations in the first voltage signal (e.g., $V_{TIA}$). For instance, this rate of adjustment determines how quickly $V_x$ aligns with $V_{TIA}$ when the feedback loop is disabled.

In some implementations, control circuit 106 may include current source 107 and capacitor 108. The term "current source" may refer to an electronic component or circuit that supplies a constant or controlled current to a load. Examples of current sources may include, without limitation, voltage-controlled current sources, current-controlled current sources, constant current sources, and/or the like. The term "capacitor" may refer to a passive electrical component that stores electrical energy in an electric field. Examples of capacitors may include, without limitation, electrolytic capacitors, ceramic capacitors, film capacitors, and/or the like.

In various examples, current source 107 provides a steady current ($I_t$) that charges or discharges capacitor 108. Capacitor 108 may be characterized by a first capacitance (e.g., $C_t$). The combination of current source 107 and capacitor 108 may be configured to define a slew rate (e.g., $I_t / C_t$) associated with the rate of adjustment of the second voltage signal. The term "slew rate" may refer to the maximum rate of change of voltage per unit of time. The slew rate represents how quickly a signal can change in response to an input or external condition. For instance, the slew rate defines the speed at which the second voltage signal can adjust in response to changes in the first voltage signal. As the feedback loop operates, the second voltage signal may gradually adjust to track the changes in the first voltage signal. The speed of this adjustment may be determined by the slew rate, ensuring that the baseline is adjusted gradually without causing abrupt transitions.

In some implementations, system 100 may include one or more comparators (e.g., comparators 109a, 109b, 109c) coupled to first amplifier 102. The term "comparator" may refer to an electronic circuit that compares two input voltages and provides a digital output based on the comparison. Examples of comparators may include, without limitation, voltage comparators, window comparators, digital comparators, and/or the like. In various examples, comparators 109a, 109b, and 109c may be used to evaluate the first voltage signal (e.g., $V_{TIA}$) against predetermined threshold voltages. The term "threshold voltage" may refer to a specific voltage level used by the comparator as a reference point to evaluate the first voltage signal. For instance, the threshold voltage defines a boundary or limit that the first voltage signal must exceed or fall below to trigger a response. When the first voltage signal exceeds or falls below the threshold set for each comparator, the comparator may generate an output signal indicating whether the signal has crossed the threshold. This allows the system to detect specific events or signal conditions based on the magnitude of the first voltage signal. For instance, first comparator 109a may be coupled to first amplifier 102 and configured to compare the first voltage signal with a first threshold voltage and generate an output signal (e.g., cmp1RAW) based on the comparison. As circuit 103 tracks and adjusts the baseline of the first voltage signal (e.g., the lower envelope of $V_{TIA}$), it ensures that the signal being fed to the comparators is free from baseline drift or long-term fluctuations. By maintaining a stable and corrected baseline, circuit 103 helps prevent false detections or misinterpretation of the signal due to baseline shifts.

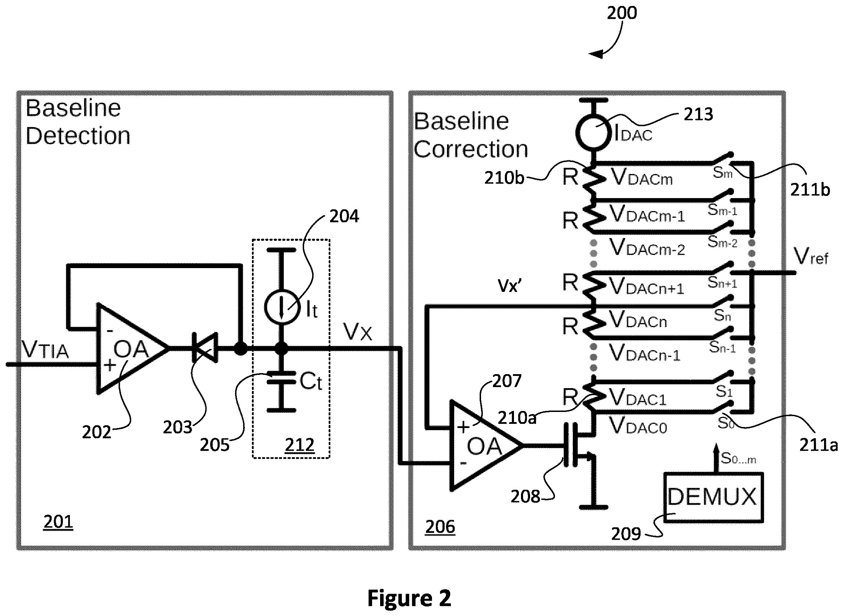

Figure 2 is a simplified diagram illustrating a system 200 for baseline detection and compensation according to embodiments of the subject technology. This diagram merely provides an example, which should not unduly limit the scope of the claims. One of ordinary skill in the art would recognize many variations, alternatives, and modifications. In various implementations, system 200 may be designed to track the baseline of an input signal and utilize the baseline information for generating precise reference voltages. The system is beneficial for applications requiring real-time baseline correction and precise event detection, such as in medical imaging (e.g., CT and PET scanners) or other sensor-based systems that rely on accurate signal processing.

According to some embodiments, system 200 may include circuit 201, which may be part of a larger system (e.g., system 100 of Figure 1) for processing sensor signals. For instance, circuit 201 may be configured to track the baseline of an input signal (e.g., $V_{TIA}$), such as the voltage signal generated by an amplifier (e.g., first amplifier 102 of Figure 1). The input signal to circuit 201 may represent the output of a sensor and include an AC component and/or a DC component. For example, the AC component may represent the photon detection events, where the intensity and timing of the signal vary based on the number and energy of the detected photons. The DC component-which may be referred to as baseline-may represent the steady-state portion of the signal when no significant external events or photon detection occurs. Circuit 201 may be configured to isolate and track the baseline, ensuring that any shifts due to external factors (e.g., temperature, aging components) are accounted for and corrected in real time.

In various implementations, circuit 203 may include first amplifier 202. For instance, first amplifier 202 may include an operational amplifier. Circuit 203 may be configured to receive a first voltage signal (e.g., $V_{TIA}$) and generate a second voltage signal (e.g., $V_x$) based on the first voltage signal. For example, the second voltage signal represents the lower envelope of the first voltage signal. In other words, circuit 201 isolates and amplifies the baseline portion of the signal, filtering out the event-driven (e.g., AC component) fluctuations, allowing the system to focus on the steady-state level that is unaffected by transient events.

In some examples, circuit 201 may further include switch 203 coupled to first amplifier 202. Switch 203 may be configured to adjust the second voltage signal in response to changes in the first voltage signal. For instance, switch 203, which may include a diode, serves as the control mechanism for the feedback loop within circuit 201. The feedback connection may be established between the output and input of first amplifier 202. In some embodiments, the feedback loop continuously compares the second voltage signal to the input signal (e.g., the first voltage signal), enabling circuit 202 to track the baseline and respond to any deviations in the first voltage signal. For instance, when the first voltage signal (e.g., $V_{TIA}$) falls below the second voltage signal (e.g., $V_x$), switch 203 may close the loop by allowing feedback to flow from the output back to the input of first amplifier 202. This feedback causes the amplifier to adjust the second voltage signal, aligning the baseline with the lower envelope of the first voltage signal. When the first voltage signal exceeds the second voltage signal, switch 203 may open and disable the feedback, preventing the baseline from being affected by event-related spikes.

According to some embodiments, circuit 201 further includes control circuit 212, which may be configured to control a rate of adjustment of the second voltage signal. For instance, this rate of adjustment determines how quickly $V_x$ aligns with $V_{TIA}$ when the feedback loop is disabled. In some examples, control circuit 212 may include first current source 204 and first capacitor 205. First current source 204 provides a steady current ($I_t$) that charges or discharges first capacitor 205. First capacitor 205 may be characterized by a first capacitance (e.g., $C_t$). The combination of first current source 204 and first capacitor 205 may be configured to define a slew rate (e.g., $I_t/C_t$) associated with the rate of adjustment of the second voltage signal. For instance, the slew rate defines the speed at which the second voltage signal can adjust in response to changes in the first voltage signal. As the feedback loop operates, the second voltage signal may gradually adjust to track the changes in the first voltage signal.

Circuit 201 provides important baseline information that ensures stability and accuracy in event detection. By maintaining a continuously adjusted baseline, circuit 201 helps prevent erroneous detections that may be caused by long-term drift or environmental fluctuations, such as temperature changes or sensor aging. The output of circuit 201 (e.g., $V_x$) serves as the baseline signal, which may be used to adjust threshold levels or reference voltages in various subsystems. For instance, circuit 201 may be coupled to a digital-to-analog converter (DAC) 206. The term "digital-to-analog converter" (DAC) may refer to an electronic circuit that converts a digital input signal into a corresponding analog output signal. Examples of DACs may include, without limitation, binary-weighted DAC, pulse-width modulator (PWM) DAC, and/or the like.

In some examples, DAC 206 may include second amplifier 207. For instance, second amplifier 207 may include an operational amplifier configured to receive the second voltage signal (e.g., the baseline signal) from circuit 201. Second amplifier 207 amplifies and processes the baseline signal to generate an output voltage that can be further processed by DAC 206. The output voltage may be used to calibrate the individual levels of DAC 206 to account for the detected baseline shifts.

In various embodiments, DAC 206 may include one or more resistors (e.g., resistors 210a, 210b), which are configured to establish a resistor network for generating different voltage levels. DAC 206 may further include one or more switches (e.g., switches 211a, 211b) coupled to one or more resistors. Each switch may be coupled to a corresponding resistor. For instance, switch 211a may be coupled to resistor 210a, and switch 211b may be coupled to resistor 210b. The resistors (e.g., resistors 210a, 210b) work in conjunction with the switches (e.g., switches 211a, 211b) to adjust the DAC's output voltages, which corresponds to the reference voltage levels needed for the signal processing system. In some cases, DAC 206 may further include demultiplexer 209, which is configured to control the resistor network by routing control signals to the appropriate switches.

In some implementations, DAC 206 may include second current source 213, which may be configured to provide a controlled current (e.g., $I_{DAC}$) to the resistor network. Depending on the implementation, the baseline signal (e.g., $V_x$) provided by circuit 201 may be used to calibrate the individual DAC levels by adjusting the reference voltage at each node in the resistor network. For example, the baseline signal may be fed into second amplifier 207, which works in conjunction with transistor 208 and the resistor network (e.g., resistors 210a, 210b) to modify the DAC's output (e.g., $V_{DAC0}$, $V_{DAC1}$, ..., $V_{DACm}$). This ensures that the threshold voltages used in the system are accurately aligned with the detected baseline. For instance, the DAC output voltages may be calculated using the following formula:

$$V_{DACk} = (V_x - n \cdot R \cdot I_{DAC}) + k \cdot R \cdot I_{DAC}$$

where $V_{DACk}$ is the output voltage at the $k^{th}$ DAC level, $V_x$ is the baseline signal voltage, $n$ is the number of resistors connected below $V_x$, R is the resistance value of the corresponding resistor, $I_{DAC}$ is the current provided by second current source 213, k is the index of the specific DAC output level being calculated.

The number of resistors in the network can be adjusted to meet the system's operational range and requirements, offering flexibility in compensating for mismatches or calibration needs. For example, the number of resistors (e.g., $m+1$) can be determined to meet specific voltage range requirements, ensuring that the impact of any mismatch on the DAC output remains within manageable limits (e.g., $n \cdot R \cdot I_{DAC}$). By incorporating the baseline signal into the calibration process, DAC 206 dynamically responds to changes in the baseline, ensuring the threshold voltages used for event detection remain stable and correctly aligned with the current baseline of the input signal.

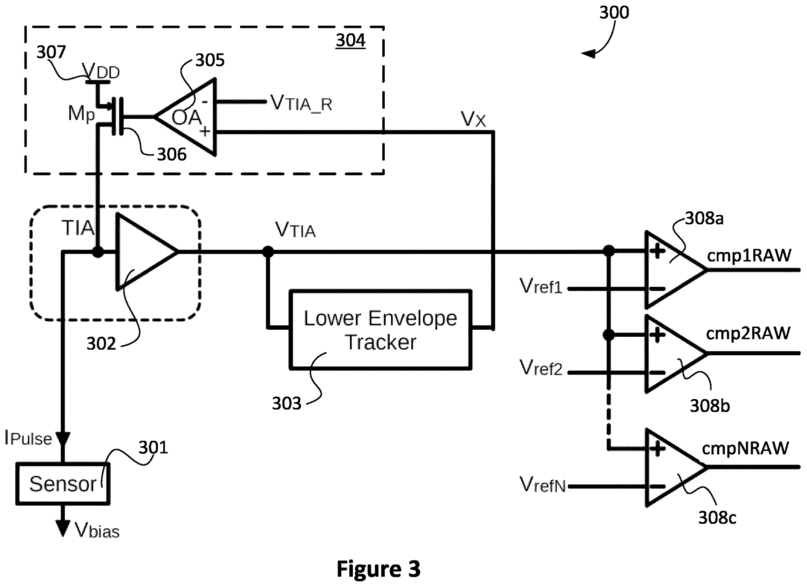

Figure 3 is a simplified diagram illustrating a front-end signal processing system 300 according to embodiments of the subject technology. This diagram merely provides an example, which should not unduly limit the scope of the claims. One of ordinary skill in the art would recognize many variations, alternatives, and modifications. In various implementations, system 300 may be part of a sensor-based system designed to receive, process, and/or interpret signals generated by various types of sensors and may be used in various applications such as medical imaging, industrial monitoring, environmental sensing, or telecommunications.

As shown, system 300 may include sensor 301. For instance, sensor 301 may include a photon detector, which is configured to detect incoming photons and convert the resulting energy into an electrical current signal (e.g., $I_{Pulse}$) that is proportional to the photon flux. This current signal may carry event-driven information, such as detecting individual photons or groups of photons in medical imaging systems.

In some examples, the output current signal of sensor 301 may include an AC component and/or a DC component. For instance, the AC component may represent the photon detection events, where the intensity and timing of the signal vary based on the number and energy of the detected photons. The DC component-which may be referred to as baseline-may represent the steady-state portion of the signal when no significant external events or photon detection occurs. The baseline value may fluctuate over time due to factors such as temperature variations, environmental changes, or component aging. The baseline serves as a reference level for event detection, and maintaining its stability is beneficial to ensure that photon events (e.g., AC component) are accurately detected and interpreted.

In some implementations, system 300 may include first amplifier 302, which may be configured to receive a current signal and generate a first voltage signal. The first voltage signal may be associated with the current signal. For instance, first amplifier 302 is configured to convert the current signal $I_{Pulse}$ generated by sensor 301 into the first voltage signal (e.g., $V_{TIA}$). The first voltage signal may be proportional to the magnitude of the current detected by sensor 301 and represent the photon flux or other physical phenomena being measured.

In various embodiments, system 300 may include first circuit 303 coupled to first amplifier 302. First circuit 303 may be configured to track and adjust the baseline of the output signal generated by first amplifier 302. For instance, first circuit 303 incrementally tracks the envelope of the first voltage signal, focusing on the portion of the signal that remains unaffected by transient events, such as photon detection or other signal fluctuations. In some examples, first circuit 303 may be configured to receive the first voltage signal (e.g., $V_{TIA}$) and generate a second voltage signal (e.g., $V_x$) based on the first voltage signal. For instance, the second voltage signal represents the lower envelope of the first voltage signal. In other words, first circuit 303 isolates and amplifies the baseline portion of the signal, filtering out event-driven (e.g., AC component) fluctuations, allowing the system to focus on the steady-state level unaffected by transient events. Circuit 303 (which may also be referred to as "lower envelope tracker") operates by continuously monitoring the output of first amplifier 302 and ensures that the baseline is continuously monitored and adjusted, preventing long-term drift due to external factors like temperature changes or aging components.

In some implementations, system 300 may include one or more comparators (e.g., comparators 308a, 308b, 308c) coupled to first amplifier 302. In various examples, comparators 309a, 309b, and 309c may be used to evaluate the first voltage signal (e.g., $V_{TIA}$) against predetermined threshold voltages. This allows the system to detect specific events or signal conditions based on the magnitude of the first voltage signal. For instance, first comparator 308a may be coupled to first amplifier 302 and configured to compare the first voltage signal with a first threshold voltage (e.g., $V_{ref1}$) and generate a first output signal (e.g., cmp1RAW) based on the comparison. Second comparator 308b may be configured to compare the first voltage signal with a second threshold voltage (e.g., $V_{ref2}$) and generate a second output signal (e.g., cmp2RAW) based on the comparison. Third comparator 308c may be configured to compare the first voltage signal with a third threshold voltage (e.g., $V_{ref3}$) and generate a third output signal (e.g., cmp3RAW) based on the comparison.

According to some embodiments, the second voltage signal (e.g., $V_x$) may be used for fine-tuning the baseline before the output signal is passed on to subsequent processing stages (e.g., comparators 308a, 308b, 308c). For instance, system 300 further includes second circuit 304 coupled to first circuit 303 and/or first amplifier 302. Second circuit 304 may utilize the second voltage signal for current subtraction, correcting any residual baseline deviation in the TIA output by injecting or removing current from the signal, ensuring that the final signal sent to the comparators is free from baseline shifts.

In some examples, second circuit 304 includes second amplifier 305, which is coupled to first circuit 303 to receive the second voltage signal. Second amplifier 305 may be configured to compare the second voltage signal (e.g., $V_x$) against a reference voltage (e.g., $V_{TIA\_R}$). The reference voltage may be used to define the desired baseline level, ensuring that any drift in the baseline is corrected before the signal is processed by the comparators. Depending on the implementation, the reference voltage may be applied externally or generated within the front end.

In some implementations, second circuit 304 further includes transistor 306, which is controlled by the output of second amplifier 305. Transistor 306 may be coupled to power supply 307 and is configured to inject a current into the input signal of first amplifier 302 (e.g., the current signal) based on the comparison between the second voltage signal and the reference voltage. Depending on the implementation, transistor 306 may include, without limitation, a PMOS transistor, an NMOS transistor, and/or the like. This current subtraction process performed by second circuit 304 helps to ensure that the final output signal is baseline-corrected, preventing long-term drift from affecting signal accuracy.

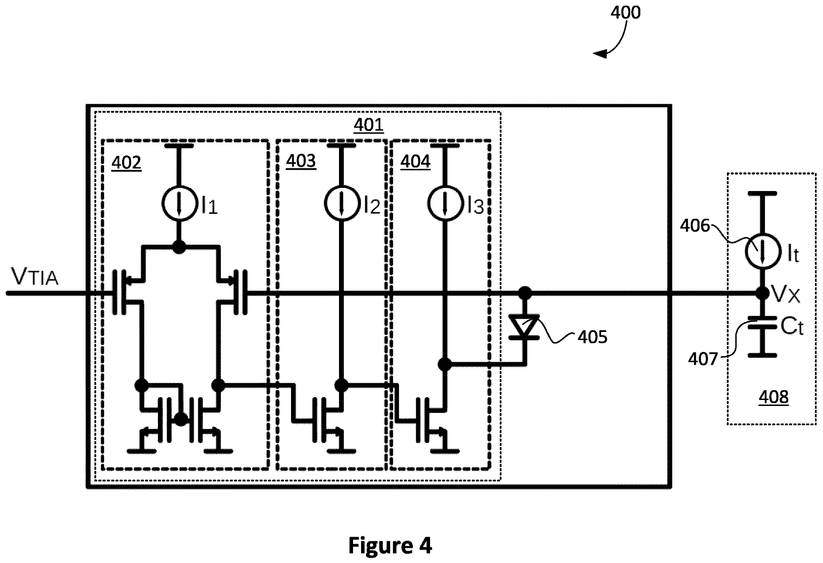

Figure 4 is a simplified diagram illustrating a system 400 for baseline detection according to embodiments of the subject technology. This diagram merely provides an example, which should not unduly limit the scope of the claims. One of ordinary skill in the art would recognize many variations, alternatives, and modifications.

In various implementations, system 400 may be part of a larger system (e.g., system 100 of Figure 1) for processing sensor signals. For instance, system 400 may be configured to track the baseline of an input signal (e.g., $V_{TIA}$), such as the voltage signal generated by an amplifier (e.g., first amplifier 102 of Figure 1). The input signal to system 400 may represent the output of a sensor and may include an AC component and/or a DC component. For example, the AC component may represent the photon detection events, where the intensity and timing of the signal vary based on the number and energy of the detected photons. The DC component-which may be referred to as baseline-may represent the steady-state portion of the signal when no significant external events or photon detection occurs. System 400 may be configured to isolate and track this baseline, ensuring that any shifts due to external factors (e.g., temperature, aging components) are accounted for and corrected in real time.

In some embodiments, system 400 may include amplifier 401. System 400 may be configured to receive a first voltage signal (e.g., $V_{TIA}$) and generate a second voltage signal (e.g., $V_x$) based on the first voltage signal. For example, the second voltage signal represents the lower envelope of the first voltage signal. In other words, system 400 isolates and amplifies the baseline portion of the signal, filtering out the event-driven (e.g., AC component) fluctuations, allowing the system to focus on the steady-state level that is unaffected by transient events.

Depending on the implementation, amplifier 401 may be configured as a single-stage or multi-stage amplifier, designed to provide the necessary gain and feedback control for baseline detection. For instance, amplifier 401 may be configured as a multi-stage amplifier and include first stage 402, second stage 403, and third stage 404. First stage 402 may include a differential pair of PMOS transistors with NMOS transistors as an active load (e.g., $I_1$), providing the initial amplification of the input signal (e.g., $V_{TIA}$). Second stage 403 and third stage 404 may include common-source amplifier stages that use single NMOS transistors with active loads (e.g., $I_2$ and $I_3$, respectively). These stages provide further amplification of the signal while maintaining high output impedance. In some implementations, a complementary configuration may be used, where the amplifier stages employ NMOS transistors as input devices and PMOS transistors as active loads, or other suitable variations, depending on design requirements.

In various implementations, system 400 may further include switch 405 coupled to amplifier 401. Switch 405 may be configured to adjust the second voltage signal in response to changes in the first voltage signal. For example, switch 405 may include a diode. In some examples, system 400 further includes control circuit 408 coupled to switch 405. Control circuit 408 may be configured to control a rate of adjustment of the second voltage signal.

In some implementations, control circuit 408 may include current source 406 and capacitor 407. Current source 406 may be configured to provide a steady current (e.g., It) that charges or discharges capacitor 407. Capacitor 407 may be characterized by a first capacitance (e.g., $C_t$). The combination of current source 406 and capacitor 407 defines a slew rate (e.g., It $/C_t$) associated with the rate of adjustment of the second voltage signal. For instance, the slew rate defines the speed at which the second voltage signal can adjust in response to changes in the first voltage signal.

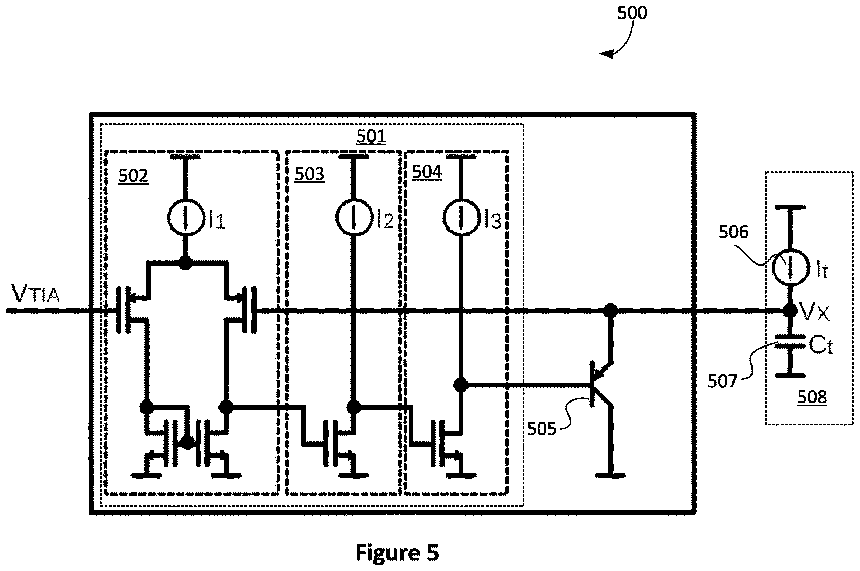

Figure 5 is a simplified diagram illustrating a system 500 for baseline detection according to embodiments of the subject technology. This diagram merely provides an example, which should not unduly limit the scope of the claims. One of ordinary skill in the art would recognize many variations, alternatives, and modifications.

In various implementations, system 500 may be part of a larger system (e.g., system 100 of Figure 1) for processing sensor signals. Similar to system 400 of Figure 4, system 500 may be configured to track the baseline of an input signal (e.g., $V_{TIA}$), such as the voltage signal generated by an amplifier (e.g., first amplifier 102 of Figure 1). The input signal to system 500 may represent the output of a sensor. For instance, the input signal to system 500 may include a first voltage signal (e.g., $V_{TIA}$), which may include an AC component and/or a DC component. The AC component may represent the photon detection events, where the intensity and timing of the signal vary based on the number and energy of the detected photons. The DC component-which may be referred to as baseline-may represent the steady-state portion of the signal when no significant external events or photon detection occurs. System 500 may be configured to isolate and track this baseline, ensuring that any shifts due to external factors (e.g., temperature, aging components) are accounted for and corrected in real time.

In various implementations, system 500 may include amplifier 501. System 500 may be configured to receive the first voltage signal (e.g., $V_{TIA}$) and generate a second voltage signal (e.g., $V_x$) based on the first voltage signal. For example, the second voltage signal represents the lower envelope of the first voltage signal. In some examples, amplifier 501 may be configured as a single-stage or multi-stage amplifier, designed to provide the necessary gain and feedback control for baseline detection. For instance, amplifier 501 may be configured as a multi-stage amplifier and include first stage 502, second stage 503, and third stage 504. First stage 502 may include a differential pair of PMOS transistors with NMOS transistors as an active load (e.g., $I_1$), providing the initial amplification of the input signal (e.g., $V_{TIA}$). Second stage 503 and third stage 504 may include common-source amplifier stages that use single NMOS transistors with active loads (e.g., $I_2$ and $I_3$, respectively). These stages provide further amplification of the signal while maintaining high output impedance.

In various implementations, system 500 may further include switch 505 coupled to amplifier 501. Switch 505 may be configured to adjust the second voltage signal in response to changes in the first voltage signal. For example, switch 505 may include a bipolar PNP transistor or an NPN transistor, which is configured to operate in diode mode, enabling current flow and feedback necessary for baseline tracking and adjustment. This approach is beneficial in metal-oxide-semiconductor field-effect transistor (MOSFET) technologies where standard diodes may not be compatible with the manufacturing process.

In some examples, system 500 further includes control circuit 508, which may be configured to control a rate of adjustment of the second voltage signal. For example, control circuit 508 may include current source 506 and capacitor 507. Current source 506 may be configured to provide a steady current (e.g., It) that charges or discharges capacitor 507. Capacitor 507 may be characterized by a first capacitance (e.g., $C_t$). The combination of current source 506 and capacitor 507 defines a slew rate (e.g., It$/C_t$) associated with the rate of adjustment of the second voltage signal. For instance, the slew rate defines the speed at which the second voltage signal can adjust in response to changes in the first voltage signal.

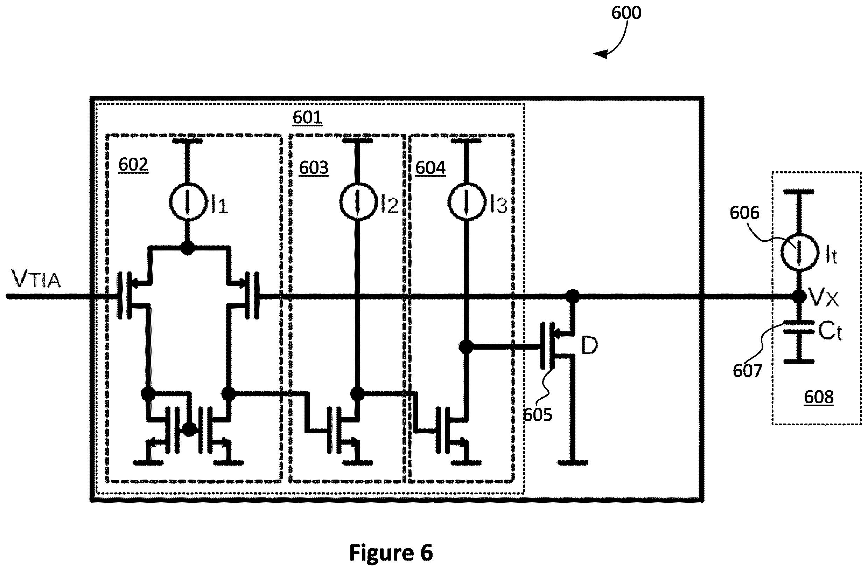

Figure 6 is a simplified diagram illustrating a system 600 for baseline detection according to embodiments of the subject technology. This diagram merely provides an example, which should not unduly limit the scope of the claims. One of ordinary skill in the art would recognize many variations, alternatives, and modifications.

In various implementations, system 600 may be part of a larger system (e.g., system 100 of Figure 1) for processing sensor signals. Similar to system 400 of Figure 4, system 600 may be configured to track the baseline of an input signal (e.g., $V_{TIA}$), such as the voltage signal generated by an amplifier (e.g., first amplifier 102 of Figure 1). The input signal to system 600 may represent the output of a sensor. For instance, the input signal to system 600 may include a first voltage signal (e.g., $V_{TIA}$), which may include an AC component and/or a DC component. The AC component may represent the photon detection events, where the intensity and timing of the signal vary based on the number and energy of the detected photons. The DC component-which may be referred to as baseline-may represent the steady-state portion of the signal when no significant external events or photon detection occurs. System 600 may be configured to isolate and track this baseline, ensuring that any shifts due to external factors (e.g., temperature, aging components) are accounted for and corrected in real time.

In various implementations, system 600 may include amplifier 601. System 600 may be configured to receive the first voltage signal (e.g., $V_{TIA}$) and generate a second voltage signal (e.g., $V_x$) based on the first voltage signal. For example, the second voltage signal represents the lower envelope of the first voltage signal. In some examples, amplifier 601 may be configured as a single-stage or multi-stage amplifier, designed to provide the necessary gain and feedback control for baseline detection. For instance, amplifier 601 may be configured as a multi-stage amplifier and include first stage 602, second stage 603, and third stage 604. First stage 602 may include a differential pair of PMOS transistors with NMOS transistors as an active load (e.g., $I_1$), providing the initial amplification of the input signal (e.g., $V_{TIA}$). Second stage 603 and third stage 604 may include common-source amplifier stages that use single NMOS transistors with active loads (e.g., $I_2$ and $I_3$, respectively). These stages provide further amplification of the signal while maintaining high output impedance.

In various implementations, system 600 may further include switch 605 coupled to amplifier 601. Switch 605 may be configured to adjust the second voltage signal in response to changes in the first voltage signal. For example, switch 605 may include a PMOS transistor. Depending on the specific implementation, other devices such as an NMOS transistor may be used as well.

In some examples, system 600 further includes control circuit 608, which may be configured to control a rate of adjustment of the second voltage signal. For example, control circuit 608 may include current source 606 and capacitor 607. Current source 606 may be configured to provide a steady current (e.g., It) that charges or discharges capacitor 607. Capacitor 607 may be characterized by a first capacitance (e.g., $C_t$). The combination of current source 606 and capacitor 607 defines a slew rate (e.g., It$/C_t$) associated with the rate of adjustment of the second voltage signal. For instance, the slew rate defines the speed at which the second voltage signal can adjust in response to changes in the first voltage signal.

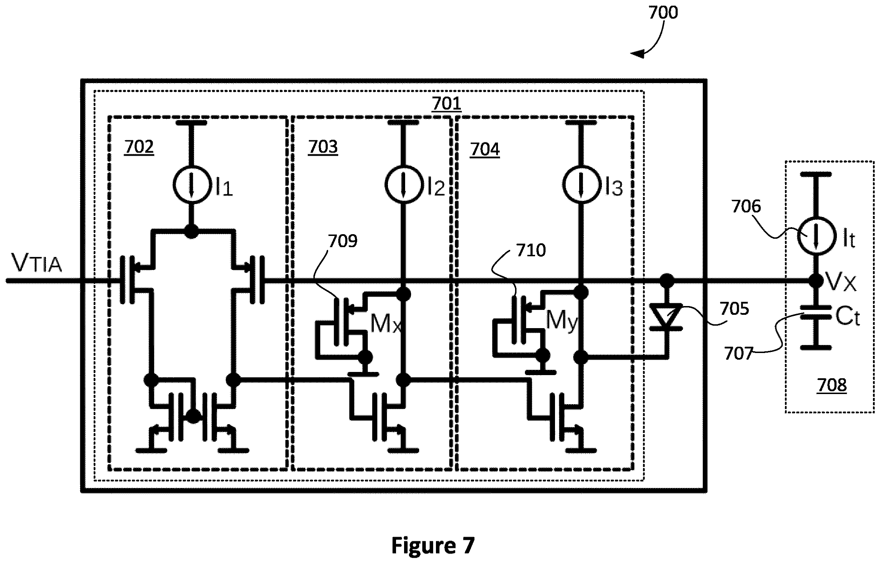

Figure 7 is a simplified diagram illustrating a system 700 for baseline detection according to embodiments of the subject technology. This diagram merely provides an example, which should not unduly limit the scope of the claims. One of ordinary skill in the art would recognize many variations, alternatives, and modifications.

In various implementations, system 700 may be part of a larger system (e.g., system 100 of Figure 1) for processing sensor signals. Similar to system 400 of Figure 4, system 700 may be configured to track the baseline of an input signal (e.g., $V_{TIA}$), such as the voltage signal generated by an amplifier (e.g., first amplifier 102 of Figure 1). The input signal to system 700 may represent the output of a sensor. For instance, the input signal to system 700 may include a first voltage signal (e.g., $V_{TIA}$), which may include an AC component and/or a DC component. The AC component may represent the photon detection events, where the intensity and timing of the signal vary based on the number and energy of the detected photons. The DC component-which may be referred to as baseline-may represent the steady-state portion of the signal when no significant external events or photon detection occurs. System 700 may be configured to isolate and track this baseline, ensuring that any shifts due to external factors (e.g., temperature, aging components) are accounted for and corrected in real time.

In various implementations, system 700 may include amplifier 701. System 700 may be configured to receive the first voltage signal (e.g., $V_{TIA}$) and generate a second voltage signal based on the first voltage signal. For example, the second voltage signal represents the lower envelope of the first voltage signal. In some examples, amplifier 701 may be configured as a single-stage or multi-stage amplifier, designed to provide the necessary gain and feedback control for baseline detection. For instance, amplifier 701 may be configured as a multi-stage amplifier and include first stage 702, second stage 703, and third stage 704. First stage 702 may include a differential pair of PMOS transistors with NMOS transistors as an active load (e.g., $I_1$), providing the initial amplification of the input signal (e.g., $V_{TIA}$). Second stage 703 and third stage 704 may include common-source amplifier stages that use single NMOS transistors with active loads (e.g., $I_2$ and $I_3$, respectively). These stages provide further amplification of the signal while maintaining high output impedance.

In various embodiments, second stage 703 may further include transistor 709 and third stage 704 may further include transistor 710. Transistors 709 and 710 may be configured to prevent the intermediate nodes from entering deep saturation. These transistors act similarly to MOSFET diodes, ensuring that the voltage at the output nodes of each stage does not cause the active loads (e.g., $I_2$ and $I_3$) to enter the deep triode region. This prevents slow response times that could occur if the active loads become saturated.

In various implementations, system 700 may further include switch 705 coupled to amplifier 701. Switch 705 may be configured to adjust the second voltage signal in response to changes in the first voltage signal. For example, switch 705 may include, without limitation, a diode, a PNP transistor, a PMOS transistor, an NMOS transistor, an NPN transistor, and/or the like. Other switching components suitable for controlling current flow and feedback may also be used depending on the circuit's requirements. In some examples, system 700 further includes control circuit 708, which may be configured to control a rate of adjustment of the second voltage signal.

In some implementations, control circuit 708 may include current source 706 and capacitor 707. Current source 706 may be configured to provide a steady current (e.g., It) that charges or discharges capacitor 707. Capacitor 707 may be characterized by a first capacitance (e.g., $C_t$). The combination of current source 706 and capacitor 707 defines a slew rate (e.g., It /Ct) associated with the rate of adjustment of the second voltage signal. For instance, the slew rate defines the speed at which the second voltage signal can adjust in response to changes in the first voltage signal.

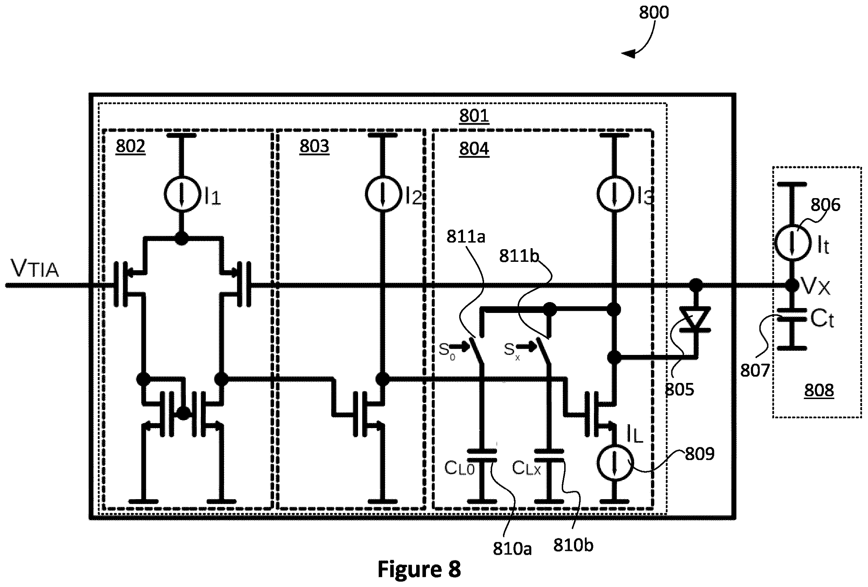

Figure 8 is a simplified diagram illustrating a system 800 for baseline detection according to embodiments of the subject technology. This diagram merely provides an example, which should not unduly limit the scope of the claims. One of ordinary skill in the art would recognize many variations, alternatives, and modifications.

In various implementations, system 800 may be part of a larger system (e.g., system 100 of Figure 1) for processing sensor signals. Similar to system 400 of Figure 4, system 800 may be configured to track the baseline of an input signal (e.g., $V_{TIA}$), such as the voltage signal generated by an amplifier (e.g., first amplifier 102 of Figure 1). The input signal to system 800 may represent the output of a sensor. For instance, the input signal to system 800 may include a first voltage signal (e.g., $V_{TIA}$), which may include an AC component and/or a DC component. The AC component may represent the photon detection events, where the intensity and timing of the signal vary based on the number and energy of the detected photons. The DC component-which may be referred to as baseline-may represent the steady-state portion of the signal when no significant external events or photon detection occurs. System 800 may be configured to isolate and track this baseline, ensuring that any shifts due to external factors (e.g., temperature, aging components) are accounted for and corrected in real time.

In various implementations, system 800 may include amplifier 801. System 800 may be configured to receive the first voltage signal (e.g., $V_{TIA}$) and generate a second voltage signal (e.g., $V_x$) based on the first voltage signal. For example, the second voltage signal represents the lower envelope of the first voltage signal. In some examples, amplifier 801 may be configured as a single-stage or multi-stage amplifier, designed to provide the necessary gain and feedback control for baseline detection. For instance, amplifier 801 may be configured as a multi-stage amplifier and include first stage 802, second stage 803, and third stage 804. First stage 802 may include a differential pair of PMOS transistors with NMOS transistors as an active load (e.g., $I_1$), providing the initial amplification of the input signal (e.g., $V_{TIA}$). Second stage 803 and third stage 804 may include common-source amplifier stages that use single NMOS transistors with active loads (e.g., $I_2$ and $I_3$, respectively). These stages provide further amplification of the signal while maintaining high output impedance.

In various implementations, system 800 may further include switch 805 coupled to amplifier 801. Switch 805 may be configured to adjust the second voltage signal in response to changes in the first voltage signal. For example, switch 805 may include a diode.

To prevent the tracking of significant undershoots in the first voltage signal (e.g., $V_{TIA}$), system 800 may include a current-limiting mechanism. For instance, amplifier 801 may further include first current source 809, which is configured to limit the peak current traversing switch 805. By limiting the current, first current source 809 controls the slew rate at the $V_x$ node, ensuring that the system responds smoothly to voltage changes without reducing the overall bandwidth of the system. This helps maintain the integrity of the baseline detection while ensuring a controlled response during signal undershoots.

In various examples, system 800 may further include a bandwidth regulation mechanism. For instance, system 800 may include one or more capacitors (e.g., capacitors 810a, 810b) coupled to the output of amplifier 801. These capacitors provide an additional load at the output of amplifier 801, allowing the bandwidth to be fine-tuned based on the implementation. These capacitors may be selectively engaged or disengaged using one or more switches (e.g., switches 811a, 811b) to fine-tune the bandwidth of the system. By adjusting the combination of capacitors and switches, system 800 can regulate the signal processing bandwidth to meet specific application requirements. The number of capacitors and switches may vary depending on the nature of the signal and the design specifications of the application.

In some implementations, system 800 further includes control circuit 808, which may be configured to control a rate of adjustment of the second voltage signal. For instance, control circuit 808 may include second current source 806 and capacitor 807. Second current source 806 may be configured to provide a steady current (e.g., It) that charges or discharges capacitor 807. Capacitor 807 may be characterized by a first capacitance (e.g., $C_t$). The combination of second current source 806 and capacitor 807 defines a slew rate (e.g., It /Ct) associated with the rate of adjustment of the second voltage signal. For instance, the slew rate defines the speed at which the second voltage signal can adjust in response to changes in the first voltage signal.

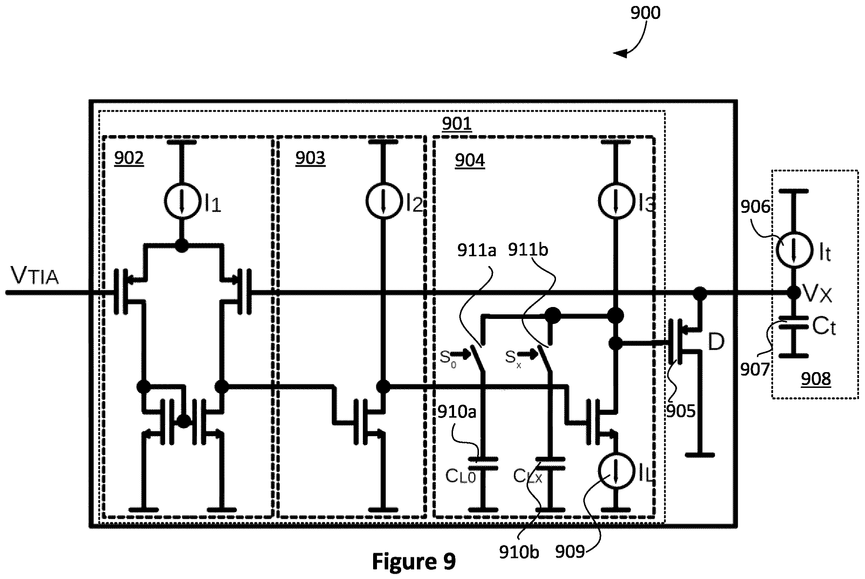

Figure 9 is a simplified diagram illustrating a system 900 for baseline detection according to embodiments of the subject technology. This diagram merely provides an example, which should not unduly limit the scope of the claims. One of ordinary skill in the art would recognize many variations, alternatives, and modifications.

In various implementations, system 900 may be part of a larger system (e.g., system 100 of Figure 1) for processing sensor signals. For instance, system 900 may be configured to track the baseline of an input signal (e.g., $V_{TIA}$), such as the voltage signal generated by an amplifier (e.g., first amplifier 102 of Figure 1). Similar to system 800 of Figure 8, system 900 may include at least one of amplifier 901, switch 905, control circuit 908, and/or the like.

Similar to amplifier 801 of Figure 8, amplifier 901 may be configured as a multi-stage amplifier and include first stage 902, second stage 903, and third stage 904. First stage 902 may include a differential pair of PMOS transistors with NMOS transistors as an active load (e.g., $I_1$), providing the initial amplification of the input signal (e.g., $V_{TIA}$). Second stage 903 and third stage 904 may include common-source amplifier stages that use single NMOS transistors with active loads (e.g., $I_2$ and $I_3$, respectively). These stages provide further amplification of the signal while maintaining high output impedance.

In some embodiments, amplifier 901 may further include first current source 909, which is configured to limit the peak current traversing switch 905 to prevent the tracking of significant undershoots in the first voltage signal (e.g., $V_{TIA}$). In various examples, system 900 may include one or more capacitors (e.g., capacitors 910a, 910b) coupled to the output of amplifier 901 through one or more switches (e.g., switches 911a, 911b). By adjusting the combination of capacitors and switches, system 900 can regulate the signal processing bandwidth to meet specific application requirements.

In various implementations, system 900 may further include switch 905 coupled to amplifier 901. Switch 905 may be configured to adjust the second voltage signal in response to changes in the first voltage signal. For example, switch 905 may include a MOSFET such as a PMOS or NMOS transistor, depending on the implementation.

In some implementations, control circuit 908 may include second current source 906 and capacitor 907. Second current source 906 may be configured to provide a steady current (e.g., It) that charges or discharges capacitor 907. Capacitor 907 may be characterized by a first capacitance (e.g., $C_t$). The combination of second current source 906 and capacitor 907 defines a slew rate (e.g., It /Ct) associated with the rate of adjustment of the second voltage signal.

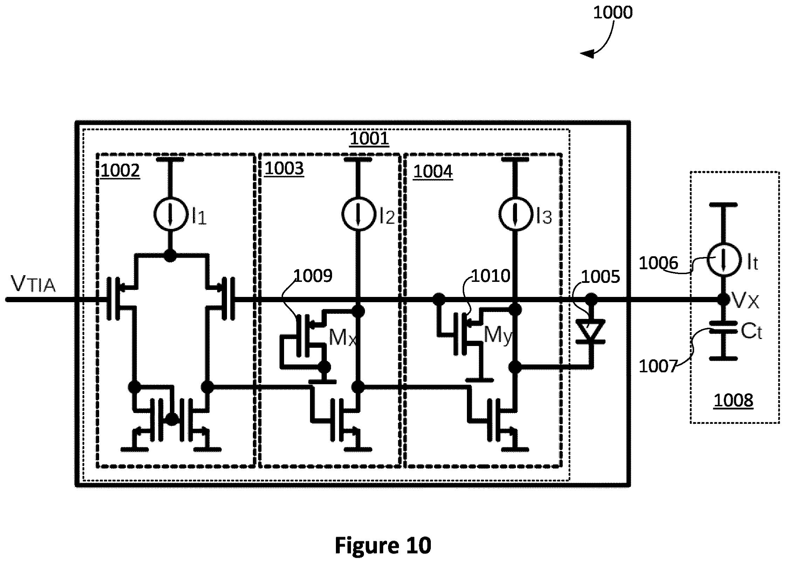

Figure 10 is a simplified diagram illustrating a system 1000 for baseline detection according to embodiments of the subject technology. This diagram merely provides an example, which should not unduly limit the scope of the claims. One of ordinary skill in the art would recognize many variations, alternatives, and modifications.

In various implementations, system 1000 may be part of a larger system (e.g., system 100 of Figure 1) for processing sensor signals. Similar to system 700 of Figure 7, system 1000 may be configured to track the baseline of an input signal (e.g., $V_{TIA}$), such as the voltage signal generated by an amplifier (e.g., first amplifier 102 of Figure 1). The input signal to system 1000 may represent the output of a sensor. For instance, the input signal to system 1000 may include a first voltage signal (e.g., $V_{TIA}$), which may include an AC component and/or a DC component. The AC component may represent the photon detection events, where the intensity and timing of the signal vary based on the number and energy of the detected photons. The DC component-which may be referred to as baseline-may represent the steady-state portion of the signal when no significant external events or photon detection occurs. System 1000 may be configured to isolate and track this baseline, ensuring that any shifts due to external factors (e.g., temperature, aging components) are accounted for and corrected in real time.

In various implementations, system 1000 may include amplifier 1001. System 1000 may be configured to receive the first voltage signal (e.g., $V_{TIA}$) and generate a second voltage signal based on the first voltage signal. For example, the second voltage signal represents the lower envelope of the first voltage signal. In some examples, amplifier 1001 may be configured as a single-stage or multi-stage amplifier, designed to provide the necessary gain and feedback control for baseline detection. For instance, amplifier 1001 may be configured as a multi-stage amplifier and include first stage 1002, second stage 1003, and third stage 1004. First stage 1002 may include a differential pair of PMOS transistors with NMOS transistors as an active load (e.g., $I_1$), providing the initial amplification of the input signal (e.g., $V_{TIA}$). Second stage 1003 and third stage 1004 may include common-source amplifier stages that use single NMOS transistors with active loads (e.g., $I_2$ and $I_3$, respectively). These stages provide further amplification of the signal while maintaining high output impedance.

In various embodiments, second stage 1003 may further include transistor 1009 and third stage 1004 may further include transistor 1010. Transistors 1009 and 1010 function to prevent the intermediate nodes from entering deep saturation. These transistors act similarly to MOSFET diodes, ensuring that the voltage at the output nodes of each stage does not cause the active loads (e.g., $I_2$ and $I_3$) to enter the deep triode region. This prevents slow response times that could occur if the active loads become saturated. In some examples, the gate of transistor 1010 may be coupled to the $V_x$ voltage node, allowing it to conduct only when $V_x$ drops below the output voltage of the third stage 1004 by more than the threshold voltage of transistor 1010. This setup ensures minimal power consumption while increasing the response time of amplifier 1001.

In various implementations, system 1000 may further include switch 1005 coupled to amplifier 1001. Switch 1005 may be configured to adjust the second voltage signal in response to changes in the first voltage signal. For example, switch 1005 may include, without limitation, a diode, a PNP transistor, a PMOS transistor, an NMOS transistor, an NPN transistor, and/or the like. Other switching components suitable for controlling current flow and feedback may also be used depending on the circuit's requirements. In some examples, system 1000 further includes control circuit 1008, which may be configured to control a rate of adjustment of the second voltage signal.

In some implementations, control circuit 1008 may include current source 1006 and capacitor 1007. Current source 1006 may be configured to provide a steady current (e.g., It) that charges or discharges capacitor 1007. Capacitor 1007 may be characterized by a first capacitance (e.g., $C_t$). The combination of current source 1006 and capacitor 1007 may be configured to define a slew rate (e.g., It /Ct) associated with the rate of adjustment of the second voltage signal. For instance, the slew rate defines the speed at which the second voltage signal can adjust in response to changes in the first voltage signal.

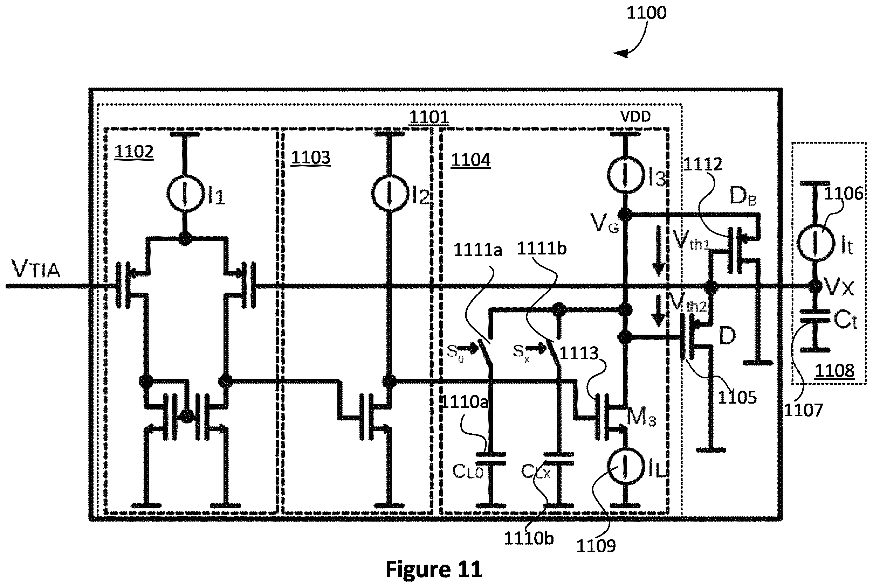

Figure 11 is a simplified diagram illustrating a system 1100 for baseline detection according to embodiments of the subject technology. This diagram merely provides an example, which should not unduly limit the scope of the claims. One of ordinary skill in the art would recognize many variations, alternatives, and modifications.

In various implementations, system 1100 may be part of a larger system (e.g., system 100 of Figure 1) for processing sensor signals. For instance, system 1100 may be configured to track the baseline of an input signal (e.g., $V_{TIA}$), such as the voltage signal generated by an amplifier (e.g., first amplifier 102 of Figure 1). Similar to system 900 of Figure 9, system 1100 may include at least one of amplifier 1101, switch 1105, control circuit 1108, and/or the like.

Similar to amplifier 901 of Figure 9, amplifier 1101 may be configured as a multi-stage amplifier and include first stage 1102, second stage 1103, and third stage 1104. First stage 1102 may include a differential pair of PMOS transistors with NMOS transistors as an active load (e.g., $I_1$), providing the initial amplification of the input signal (e.g., $V_{TIA}$). Second stage 1103 and third stage 1104 may include common-source amplifier stages that use single NMOS transistors with active loads (e.g., $I_2$ and $I_3$, respectively). These stages provide further amplification of the signal while maintaining high output impedance.

In some embodiments, amplifier 1101 may further include first current source 1109, which is configured to limit the peak current to prevent the tracking of significant undershoots in the first voltage signal (e.g., $V_{TIA}$). In various examples, system 1100 may include one or more capacitors, such as capacitor 1110a (e.g., $C_{L0}$) and capacitor 1110b ($C_{LX}$), which are coupled to the output of amplifier 1101 through one or more switches (e.g., switches 1111a, 1111b). By adjusting the combination of capacitors and switches, system 1100 can regulate the signal processing bandwidth to meet specific application requirements.

In various implementations, system 1100 may further include switch 1105 coupled to amplifier 1101. For example, switch 1105 may include, without limitation, a diode, a PNP transistor, a PMOS transistor, an NMOS transistor, an NPN transistor, and/or the like. Other switching components suitable for controlling current flow and feedback may also be used depending on the circuit's requirements.

In some implementations, control circuit 1108 may include second current source 1106 and capacitor 1107. Second current source 1106 may be configured to provide a steady current (e.g., It) that charges or discharges capacitor 1107. Capacitor 1107 may be characterized by a first capacitance (e.g., $C_t$). The combination of second current source 1106 and capacitor 1107 defines a slew rate (e.g., It /Ct) associated with the rate of adjustment of the second voltage signal.

In various embodiments, an improvement in system 1100 is the addition of a transistor 1112 (e.g., $D_B$), which serves to prevent unpredictable timing behavior when the feedback loop is inactive. Transistor 1112 may be coupled to switch 1105. When amplifier 1101 is in idle mode, and $V_{TIA}$ is higher than $V_X$, current source $I_3$ may inject current into the node $V_G$. In previous designs (e.g., as shown in Figure 9), the voltage at the $V_G$ node would rise close to the supply voltage (e.g., VDD), resulting in an uncontrolled voltage difference between the $V_G$ and $V_X$ nodes. This could lead to erratic behavior when the circuit attempts to follow sudden voltage dips, often caused by crosstalk in pixelated detectors.

To address this, transistor 1112 is introduced to clamp the $V_G$ node, preventing it from rising too far above $V_X$. For example, transistor 1112 limits the maximum voltage at $V_G$ node by diverting current from $I_3$ to ground, thereby capping $V_G$ at $V_X + V_{th1}$ (where Vth1 may be the threshold voltage of transistor 1112). This ensures that $V_G$ follows $V_X$ more closely, maintaining a predictable voltage difference between the two nodes.

When the input signal $V_{TIA}$ decreases and falls below the voltage at $V_X$, transistor 1113 (e.g., M3) switches on, initiating a controlled discharge of $V_G$ at a rate determined by current provided by first current source 1109 (e.g., IL) and the total effective capacitance (e.g., $C_{eff}$), which is determined by the combination of capacitors (e.g., $C_{L0}$ through $C_{LX}$) selected by switches $S_0$ through $S_X$ (e.g., switches 1111a through 1111b). The $V_G$ voltage drops from the initial level of $V_X + V_{th1}$ down to $V_X - V_{th2}$ (where $V_{th2}$ may be the threshold voltage of switch 1105). As $V_G$ drops, switch 1105 eventually turns on, allowing the system to re-establish feedback control.

The combination of these components allows system 1100 to introduce a delay before the feedback is applied, enabling the system to filter out short, transient voltage dips that might arise from cross-talk effects in pixelated detectors. For instance, transistor 1112 may be configured to delay the adjustment of the second voltage signal by a time interval. The delay introduced may be calculated as follows:

$$Delay = C_{eff} \cdot (V_{th1} + V_{th2)}/IL$$

where $C_{eff}$ represents the effective capacitance selected by the switches, $V_{th1}$ and $V_{th2}$ are the two threshold voltages defined by transistor 1112 and switch 1105, and IL is the current defined by first current source 1109. This ensures that a certain delay time must elapse before feedback is re-established, providing the system with the ability to ignore transient voltage dips that could otherwise affect baseline tracking.

This approach to baseline extraction and feedback delay is particularly beneficial in pixelated detectors, where cross-talk between neighboring pixels can cause momentary voltage fluctuations. By introducing a controlled delay, system 1100 can suppress such cross-talk effects and ensure stable and accurate baseline extraction, improving the overall signal integrity for applications such as medical imaging and other sensor-based systems.

While the above is a full description of the specific embodiments, various modifications, alternative constructions and equivalents may be used. Therefore, the above description and illustrations should not be taken as limiting the scope of the subject technology which is defined by the appended claims.

Claims

1. An apparatus, comprising:

a first amplifier configured to receive a current signal and generate a first voltage signal, the first voltage signal being associated with the current signal; and
a first circuit coupled to the first amplifier, the first circuit being configured to receive the first voltage signal and generate a second voltage signal based on the first voltage signal, the first circuit comprising:

a second amplifier coupled to the first amplifier;

a switch coupled to the second amplifier, the switch being configured to adjust the second voltage signal in response to changes in the first voltage signal; and

a control circuit coupled to the switch, the control circuit being configured to control a rate of adjustment of the second voltage signal.

2. The apparatus of claim 1, wherein

the second amplifier comprises a first input and a second input, the first input is configured to receive the first voltage signal, and the second input is configured to receive the second voltage signal.

3. The apparatus of claim 1 or 2, wherein the switch comprises a metal-oxide-semiconductor field-effect transistor. MOSFET.

4. The apparatus of any one of the claims 1 to 3, wherein

the switch comprises a diode.

5. The apparatus of any one of the claims 1 to 4, wherein

the control circuit comprises a capacitor and a current source, the capacitor and current source are configured to define a slew rate associated with the rate of adjustment of the second voltage signal.

6. The apparatus of any one of the claims 1 to 5, further comprising

a digital-to-analog converter (DAC) coupled to the first circuit, the DAC being configured to receive the second voltage signal and generate a first reference voltage;

wherein in particular

the DAC is configured to adjust the first reference voltage based on the second voltage signal.

7. The apparatus of any one of the claims 1 to 6, further comprising a second circuit coupled to the first amplifier and the first circuit, the second circuit being configured to adjust the second voltage signal based at least on a second reference voltage;

wherein in particular

the second circuit comprises a transistor configured to inject a current into the current signal.

8. The apparatus of any one of the claims 1 to 7, further comprising

a first comparator coupled to the first amplifier, the first comparator being configured to compare the first voltage signal with a first threshold voltage and generate an output signal based on the comparison.

9. An apparatus, comprising:

a first amplifier configured to receive a current signal and generate a first voltage signal, the first voltage signal being associated with the current signal; and

a first circuit coupled to the first amplifier, the first circuit being configured to generate a second voltage signal based on the first voltage signal, the first circuit comprising:

a second amplifier coupled to the first amplifier;

a switch coupled to the second amplifier, the switch being configured to adjust the second voltage signal in response to changes in the first voltage signal; and

a control circuit coupled to the switch, the control circuit being configured to control a rate of adjustment of the second voltage signal.

10. The apparatus of claim 9, wherein

the second amplifier comprises a first input and a second input, the first input is configured to receive the first voltage signal, and the second input is configured to receive the second voltage signal;

wherein in particular

the switch comprises a metal-oxide-semiconductor field-effect transistor (MOSFET).

11. The apparatus of claim 9 or 10, wherein the switch comprises a diode.

12. The apparatus of any one of the claims 9 to 11, wherein

the control circuit comprises a capacitor and a current source, the capacitor and current source are configured to define a slew rate associated with the rate of adjustment of the second voltage signal.

13. An apparatus, comprising:

a first amplifier configured to generate a first voltage signal; and

a first circuit coupled to the first amplifier, the first circuit being configured to generate a second voltage signal based on the first voltage signal, the first circuit comprising:

a second amplifier coupled to the first amplifier;

a switch coupled to the second amplifier, the switch being configured to adjust the second voltage signal in response to changes in the first voltage signal; and
a control circuit coupled to the switch, the control circuit being configured to control a rate of adjustment of the second voltage signal.

14. The apparatus of claim 13, further comprising a first comparator coupled to the first amplifier, the first comparator being configured to compare the first voltage signal with a first threshold voltage and generate an output signal based on the comparison.

15. The apparatus of claim 13 or 14, wherein the apparatus comprises at least one of the following features:

the switch comprises a diode;
the second amplifier comprises a transistor coupled to the switch, the transistor is configured to delay the adjustment of the second voltage signal by a time interval; and
the control circuit comprises a capacitor and a current source, the capacitor and current source are configured to define a slew rate associated with the rate of adjustment of the second voltage signal.

**Figure 1**

Figure 2

EP 4 718 124 A1

**Figure 3**

$V_{TIA}$

402  401  403  404

I1  I2  I3

405

It  Vx  Ct

406  407  408

400

**Figure 4**

**Figure 5**

**Figure 6**

**Figure 7**

**Figure 8**

**Figure 9**

EP 4 718 124 A1

**Figure 10**

EP 4 718 124 A1

Figure 11

Europäisches
Patentamt
European
Patent Office
Office européen
des brevets

# EUROPEAN SEARCH REPORT

**Application Number**

EP 25 20 4327

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/400547 A1 (YAMAMOTO MASANOBU [US] ET AL) 24 December 2020 (2020-12-24) <br><br> * paragraph [0003] - paragraph [0097]; figures 10a, 11a, 11c, 12a * <br> ----- | 1-4, 6-11,13, 14 | INV. <br> G01T7/00 <br> G05F1/46 <br> G05F1/56 <br> G05F1/565 <br> G05F1/575 |
| X | US 2022/321809 A1 (MYERS CHARLES [US] ET AL) 6 October 2022 (2022-10-06) <br> * paragraph [0002] - paragraph [0066]; figures 3,5,6,7 * <br> ----- | 1,2,5-9, 11-15 | G05F3/26 <br> H03G3/30 |
| X | US 2002/009109 A1 (ASANO HIROAKI [JP]) 24 January 2002 (2002-01-24) <br> * paragraph [0002] - paragraph [0045]; figures 2,4 * <br> ----- | 1-5,7, 9-13,15 | |
| X | US 2010/172467 A1 (STEADMAN BOOKER ROGER [DE] ET AL) 8 July 2010 (2010-07-08) <br> * paragraph [0001] - paragraph [0080]; figure 3 * <br> ----- | 1-5,7, 9-13,15 | |

**TECHNICAL FIELDS SEARCHED (IPC)**

G01T
H03F
G01V
G05F
H03G
G01J

The present search report has been drawn up for all claims

| Place of search | Date of completion of the search | Examiner |
|---|---|---|
| The Hague | 16 January 2026 | Cuk, Vladimir |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

.......................................................................................................

& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 25 20 4327

This annex lists the patent family members relating to the patent documents cited in the above-mentioned European search report.
The members are as contained in the European Patent Office EDP file on
The European Patent Office is in no way liable for these particulars which are merely given for the purpose of information.

16-01-2026

| Patent document cited in search report | | Publication date | Patent family member(s) | | Publication date |
|---|---|---|---|---|---|
| US 2020400547 | A1 | 24-12-2020 | US | 2020400547 A1 | 24-12-2020 |
| | | | US | 2022146401 A1 | 12-05-2022 |
| | | | US | 2024035951 A1 | 01-02-2024 |
| | | | US | 2025130156 A1 | 24-04-2025 |
| US 2022321809 | A1 | 06-10-2022 | EP | 4268365 A1 | 01-11-2023 |
| | | | TW | 202249418 A | 16-12-2022 |
| | | | TW | 202349858 A | 16-12-2023 |
| | | | US | 2022321809 A1 | 06-10-2022 |
| | | | US | 2022321810 A1 | 06-10-2022 |
| | | | US | 2022321811 A1 | 06-10-2022 |
| | | | WO | 2022212333 A1 | 06-10-2022 |
| US 2002009109 | A1 | 24-01-2002 | EP | 1162707 A2 | 12-12-2001 |
| | | | JP | 2001352125 A | 21-12-2001 |
| | | | US | 2002009109 A1 | 24-01-2002 |
| US 2010172467 | A1 | 08-07-2010 | CN | 101779144 A | 14-07-2010 |
| | | | EP | 2176684 A2 | 21-04-2010 |
| | | | US | 2010172467 A1 | 08-07-2010 |
| | | | WO | 2009019622 A2 | 12-02-2009 |

EPO FORM P0459